# EUROPÄISCHE PATENTSCHRIFT

(11) **EP 3 695 793 B1**
(45) Veröffentlichungstag und Bekanntmachung des Hinweises auf die Patenterteilung: **25.08.2021**
(21) Anmeldenummer: 20155030.8
(22) Anmeldetag: 31.01.2020
(51) Int. Cl.: A61B 17/29, A61B 17/00

(54) **MEDIZINISCHES INSTRUMENT**
MEDICAL INSTRUMENT
INSTRUMENT MÉDICAL

(30) Priorität: 12.02.2019 DE 102019103493
(43) Veröffentlichungstag der Anmeldung: 19.08.2020
(73) Patentinhaber: Karl Storz SE & Co. KG, 78532 Tuttlingen (DE)
(72) Erfinder: Hoffmann, Martin, 78532 Tuttlingen (DE); Forster, Jonas, 78532 Tuttlingen (DE); Albrecht, Simon, 10551 Berlin (DE); Böse, Gordon, 10317 Berlin (DE)
(74) Vertreter: Hofmeister, Frank

(56) Entgegenhaltungen:
- EP-A1- 2 413 819
- DE-A1-102015 015 655
- US-A1- 2004 225 323
- US-A1- 2009 320 637
- US-A1- 2016 242 800
- US-A1- 2017 120 457

## Beschreibung

Die Erfindung betrifft ein medizinisches Instrument mit einem hohlen Schaft, an dessen proximalem Ende eine Handhabe angeordnet ist und an dessen distalem Ende ein Werkzeug mit zwei Maulteilen angeordnet ist, von denen mindestens ein Maulteil relativ zu dem anderen Maulteilen verschwenkbar ist, wobei ein das Werkzeug tragender distaler Endbereich des Schaftes als gegenüber der Längsachse des Schaftes abwinkelbare Werkzeugspitze ausgebildet ist sowie die Werkzeugspitze um die Längsachse des Schaftes bzw. um die Längsachse der Werkzeugspitze drehbar ist und wobei das mindestens eine verschwenkbare Maulteil des Werkzeugs zwischen einer geschlossenen und einer geöffneten Position verstellbar ist, wobei das Drehen der Werkzeugspitze und das Betätigen der Maulteile über im Schaft gelagerte Betätigungselemente erfolgt, die proximalseitig mit der Handhabe gekoppelt sind.

Handgeführte chirurgische Instrumente für minimalinvasive Operationen die dem Operateur eine Mehrzahl an Freiheitsgraden zur Betätigung des Instrumentenkopfs bzw. der Werkzeugspitze ermöglichen, sind in verschiedenen Ausführungsformen aus der Praxis bekannt. Alle diese Instrumente versuchen bei größtmöglicher Steifigkeit in den Außenabmessungen so klein wie möglich zu bauen. Da die Instrumente zur Verwendung in der endoskopischen Chirurgie sehr klein sein müssen, aber gleichzeitig sehr kräftig zugreifen oder Schneiden müssen, ist die Belastung auf die Gelenke und die zugehörigen Ansteuermechanismen sehr groß.

Die bekannten handgeführten gelenkigen Instrumente weisen meist eine Kinematik auf, die stark auf die jeweils zu lösende Aufgabe zugeschnitten ist. Um die benötigten Freiheitsgrade für die Aufgaben im Instrumentenkopf bzw. in der Werkzeugspitze realisieren zu können, weisen diese bekannten Instrumente meist einen größeren Schaftdurchmesser auf als nicht gelenkige Instrumente.

Um bei abwinkelbaren Instrumenten ausreichende Kräfte übertragen zu können, ist es bei den aus der Praxis bekannten Instrumenten bekannt, Seilzüge oder Kegelradgetriebe zu verwenden. Bei Seilzügen, die gut dazu geeignet sind, ausreichend große Kräfte zu übertragen, kommt es immer wieder vor, dass sich organisches Material aus dem Operationsgebiet zwischen den einzelnen Adern der Seilzüge festsetzt und in den Schaft gezogen wird. Diese Verunreinigungen der Seilzüge sind beim Reinigen der Instrumente nur sehr schwer wieder zu entfernen. Darüber hinaus sind die Seilzüge bei den kleinen erforderlichen Biegeradien in den Instrumentenköpfen nicht dauerfest, wodurch die Nutzungsdauer dieser Instrumente nur eingeschränkt ist.

Die Instrumente mit Kegelradgetriebe sind in der Regel sehr gelenkig, gut reinigbar und dauerfest, jedoch ist die Leistungsdichte nicht so hoch wie bei Instrumenten mit Seilzügen, weshalb entweder die maximal übertragbare Kraft gering ist, oder aber das Instrument einen größeren Außendurchmesser aufweisen muss.

Ein gattungsgemäßes medizinisches Instrument ist aus der US 2009/0320637 A1 bekannt. Das Abwinkeln der Werkzeugspitze erfolgt bei diesem bekannten Instrument über ein als Kulissenelement ausgebildetes distales Ende des Schaftes. Diese bekannte konstruktive Ausgestaltung erzeugt in der Regel im Antriebsstrang für die Werkzeugspitze einen zu kompensierenden Längenversatz und ermöglicht keine direkte feinfühlige Kraftübertragung entlang des Instruments.

Ein weiteres medizinisches Instrument mit einer abwinkelbaren Werkzeugspitze ist aus der EP 2 413 819 A1 bekannt. Das Verschwenken der Werkzeugspitze erfolgt durch ein einfaches abklappen um eine quer zur Längsachse verlaufende Schwenkachse, wodurch ebenfalls ein Längenversatz im Antriebsstrang verursacht wird. Zur Weiterleitung der Bewegung zum Drehen der Werkzeugspitze und zum Betätigen der Maulteile dienen zwei in Reihe geschalteten identischen Kardangelenke.

Die US 2016/0242800 A1 offenbart ein zangenartiges medizinisches Instrument, dessen mit einem Werkzeug ausgestattete Werkzeugspitze gegenüber dem Instrumentenschaft verschwenkbar ist. Das Verschwenken der Werkzeugspitze erfolgt bei diesem bekannten Instrument mittels zweier aufeinander abrollender Zahnräder. Das Betätigen der Maulteile des Werkzeugs über ein mit der Handhabe verbundenes Kabel, das außen an den aufeinander abrollenden Zahnrädern entlang zum Werkzeug geführt wird.

Aus der US 2017/0120457 A1 ist mechanisches Gelenk für medizinische Instrumente bekannt, bei dem eine die Maulteile tragende Werkzeugspitze über ein Gelenk gegenüber der Längsachse des Schaftes verschwenkbar ist, wobei das Gelenk aus jeweils zwei aufeinander abrollenden Zahnrädern mit Zähnen am distalen Ende des Schaftes und am proximalen Ende der Werkzeugspitze besteht. In der Mitte zwischen den beiden Zahnrädern verbleibt eine Öffnung zum Hindurchführen eines Antriebsstrangs zum Betätigen der Werkzeuge der Werkzeugspitze.

Weitere Detaillösungen zur Ausgestaltung medizinischer Instrumente mit einer verschwenkbaren Werkzeugspitze sind aus der DE 10 2015 015 655 A1 mit zwei miteinander kämmenden Stirnverzahnungen und aus der US 2004/0225323 A1 mit einem Kardangelenk zum Betätigen des Werkzeugs der Werkzeugspitze bekannt.

Davon ausgehend liegt der Erfindung die **Aufgabe** zugrunde, ein medizinisches Instrument der eingangs genannten Art zu schaffen, dessen Gelenke bei möglichst vielen Freiheitsgraden und kleinem Bauraum eine hohe Kraftübertragung gewährleisten und dabei auch noch gut reinigbar sind.

Die **Lösung** dieser Aufgabenstellung ist erfindungsgemäß dadurch gekennzeichnet, dass zur Ausbildung eines Gelenks zum Abwinkeln der Werkzeugspitze das distale Ende des Schaftes und das proximale Ende der Werkzeugspitze kreissegmentförmig ausgebildet sind und die beiden Kreissegmente zum Abwinkeln der Werkzeugspitze aufeinander abrollen und, dass die Weiterleitung der Bewegungen der beiden Betätigungselemente zum Drehen der Werkzeugspitze und zum Betätigen der Maulteile vom Schaft in die abwinkelbare Werkzeugspitze über mindestens zwei in Reihe hintereinander geschaltete identische Gelenke erfolgt, die sowohl Dreh- als auch Schubbewegungen übertragen können, wobei die mindestens zwei in Reihe hintereinander geschalteten Gelenke als Gleichlaufgelenke ausgebildet sind.

Durch die Ausbildung des Gelenks zum Abwinkeln der Werkzeugspitze als aufeinander abrollende Kreissegmente ist es möglich, dass im Antriebsstrang für die Werkzeugspitze kein zu kompensierender Längenversatz auftritt, durch den ansonsten beim Abwinkeln der Werkzeugspitze beispielsweise eine zwangsläufige Betätigung der Maulteile des Werkzeugs auftritt.

Die Ausbildung des Antriebsstrangs zur Weiterleitung der Bewegungen der beiden Betätigungselemente zum Drehen der Werkzeugspitze und zum Betätigen der Maulteile vom Schaft in die abwinkelbare Werkzeugspitze als mindestens zwei in Reihe hintereinander geschaltete identische Gelenke ermöglicht es, die mindestens zwei Gelenke jeweils klein zu dimensionieren und aufgrund der Reihenschaltung die gewünschte hohe Gelenkigkeit zu gewährleisten.

Homokinetische oder auch gleichlaufende Gelenke zeichnen sich dadurch aus, dass sie eine gleichmäßige Winkelgeschwindigkeits- und Drehmomentübertragung von einer Welle auf eine winklig dazu angebrachte Welle ermöglichen. Die Gleichlaugelenke bestehen aus einer Kugelpfanne und einem Kugelkopf, der in der Kugelpfanne sitzt. Beide Bauteile weisen bis zu sechs Nuten auf, die axial entlang der Kugeloberflächen von Kugelpfanne und Kugelkopf ausgebildet sind, wobei jeweils eine Nut des Kugelkopfes und eine Nut der Kugelpfanne ein Nutenpaar bilden, in welchem eine Kugel zwangsgeführt wird.

Jedes Gleichlaufgelenk für sich ermöglicht die Übertragung eines Torsionsmoments bei einer Abwinklung zwischen Antriebs- und Abtriebsachse von bis zu 45°. Durch die erfindungsgemäße Reihenschaltung von mindestens zwei identischen Gleichlaufgelenken ist somit eine Abwinklung von mindestens 90° erzielbar. Axiale Kräfte sind mit den üblichen Gleichlaufgelenken aufgrund der in Aixalrichtung verlaufenden Nuten der Kugelbahnen nur in geringem Maße übertragbar.

Um die Übertragung von Axialkräften mittels eines Gleichlaufgelenks deutlich zu verbessern, wird erfindungsgemäß vorgeschlagen, dass ein Kugelkopf und eine Kugelpfanne eines jeden Gleichlaufgelenks über einen im Inneren des Gleichlaufgelenks angeordneten, in den Gelenkwellen gelagerten Draht miteinander verbunden sind. Durch den das Gelenk intern überbrückenden Draht werden die Kugelpfanne und der Kugelkopf so zueinander fixiert, dass ohne Einschränkung der Beweglichkeit des Gleichlaufgelenks nunmehr auch hohe axiale Kräfte mittels eines solchermaßen modifizierten Gleichlaufgelenks übertragbar sind.

Gemäß einer praktischen Ausführungsform zur Ausbildung dieses Axialkräfte übertragenden Gleichlaufgelenks wird mit der Erfindung vorgeschlagen, dass eine axiale Bohrung in der Kugelpfanne und im Kugelkopf den Draht führt, wobei der Draht mit beiden Komponenten, Kugelpfanne und Kugelkopf verbunden ist.

Schließlich wird mit der Erfindung vorgeschlagen, dass der Draht als Nitinoldraht ausgebildet ist. Bei dem Werkstoff Nitinol handelt es sich um eine Formgedächtnis-Legierung, die nach einer Verformung durch Erwärmung selbsttätig wieder zurück in ihre alte Form überführbar ist.

Gemäß einer praktischen Ausführungsform der Erfindung wird vorgeschlagen, dass die beiden Kreissegmente als aufeinander abrollende Zahnradprofile ausgebildet sind. Die Ausgestaltung als aufeinander abrollende Zahnradprofile stellt eine besonders einfache konstruktive Lösung dar, die es aufgrund des zwischen den beiderseitigen Zähnen auftretenden Form- und Reibschlusses ermöglicht, die Werkzeugspitze in den unterschiedlichsten abgewinkelten Positionen zu halten.

Zur Ausbildung der Zahnprofile wird gemäß einer bevorzugten Ausführungsform der Erfindung vorgeschlagen, dass das distale Ende des Schaftes und das proximale Ende der Werkzeugspitze jeweils zwei parallel und mit Abstand zueinander angeordnete Stege aufweisend gabelförmig ausgebildet sind, wobei die Zahnradprofile an den freien Enden der Stege des Schaftes und der Werkzeugspitze ausgebildet sind. Durch die Verlagerung der das Gelenk zum Abwinkeln der Werkzeugspitze bildenden Zahnprofile auf die seitlichen Stege der gabelförmigen Enden verbleibt in der Mitte des Schaftes und der Werkzeugspitze ausreichend Platz für die Durchführung des Antriebsstrangs für die Werkzeugspitze.

Weiterhin wird mit der Erfindung vorgeschlagen, dass das distale Ende des Schaftes und das proximale Ende der Werkzeugspitze über zwei Laschen miteinander verbunden sind, die in den Mittelpunkten der Zahnradprofile an den Stegen festgelegt sind. Die beiden Laschen halten die beiderseitigen Zahnradprofile in konstantem Abstand zueinander und gewährleisten so ein gleichmäßiges und im Wesentlichen spielfreies Abrollen der Zahnradprofile aufeinander beim Abwinkeln der Werkzeugspitze.

Das Abwinkeln der Werkzeugspitze gegenüber der Längsachse des Schaftes wird erfindungsgemäß über zwei im Schaft geführte und distalseitig an der Werkzeugspitze festgelegte Zug-/Schubelemente aktuiert, die vorteilhafterweise als rechteckige Nitinolbänder ausgebildet sind. Die glatten Oberflächen der Bänder lassen sich gut reinigen und nutzen aufgrund ihrer rechteckigen Form den zur Verfügung stehenden Bauraum besser aus als kreisförmige Elemente, wie beispielsweise Seilzüge.

Weitere Merkmale und Vorteile der Erfindung ergeben sich anhand der zugehörigen Zeichnungen, in denen zwei Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine perspektivische Seitenansicht eines erfindungsgemäßen medizinischen Instruments;
- Fig. 2: eine Seitenansicht des Details II gemäß Fig. 1, eine Werkzeugspitze in verschiedenen Positionen darstellend;
- Fig. 3: eine schematische Darstellung eines Gelenks zum Abwinkeln der Werkzeugspitze;
- Fig. 4: eine perspektivische und teilweise geschnittene Darstellung der Werkzeugspitze gemäß Fig. 2;
- Fig. 5: eine perspektivische teilweise als Explosionszeichnung ausgebildete Darstellung der Werkzeugspitze gemäß Fig. 4;
- Fig. 6: eine perspektivische Ansicht einer ersten Ausführungsform zur Ausbildung der Gelenke des Antriebsstrangs zum Betätigen der Werkzeugspitze, die nicht Gegenstand der Erfindung ist;
- Fig. 7: eine perspektivische Ansicht zweier in Reihe geschalteter Gelenke gemäß Fig. 6;
- Fig. 8: eine perspektivische Ansicht einer zweiten Ausführungsform zur Ausbildung der Gelenke des Antriebsstrangs zum Betätigen der Werkzeugspitze und
- Fig. 9: eine perspektivische Explosionszeichnung der Darstellung gemäß Fig. 8.

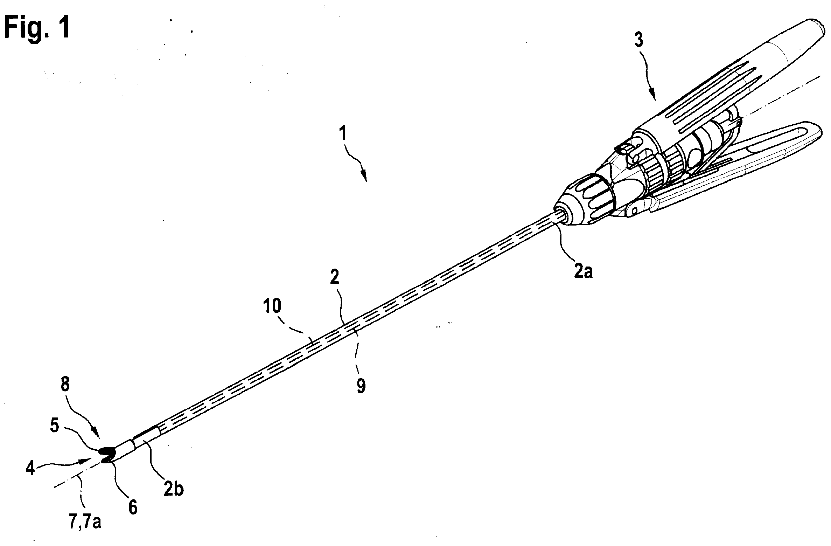

Die Abbildung Fig. 1 zeigt ein medizinisches Instrument 1 mit einem hohlen Schaft 2, an dessen proximalem Ende 2a eine Handhabe 3 angeordnet ist und an dessen distalem Ende 2b ein Werkzeug 4 angeordnet ist, das aus zwei Maulteilen 5 und 6 besteht, die bei der nachstehend näher beschriebenen Ausführungsform als relativ zueinander verschwenkbare Maulteile 5 und 6 ausgebildet sind.

Um dem Werkzeug 4 möglichst viele Freiheitsgrade zur Bewegung relativ zum Schaft 2 einzuräumen, ist ein das Werkzeug 4 tragender distaler Endbereich des Schaftes 2 als gegenüber der Längsachse 7 des Schaftes 2 abwinkelbare Werkzeugspitze 8 ausgebildet. Weiterhin ist das Werkzeug 4 um die Längsachse 7 des Schaftes 2 bzw. bei abgewinkelter Werkzeugspitze 8 um die Längsachse 7a der Werkzeugspitze 8 drehbar.

Zum Verschwenken der Maulteile 5 und 6 des Werkzeugs 4 relativ zueinander zwischen einer geschlossenen und einer geöffneten Position ist im Schaft 2 ein axial verschiebbares erstes Betätigungselement 9 gelagert, das proximalseitig mit der Handhabe 3 in Wirkverbindung steht.

Das Verdrehen der Werkzeugspitze 8 um die Längsachse 7 des Schaftes 2 bzw. der Längsachse 7a der Werkzeugspitze 8 erfolgt über ein in dem hohlen Schaft 2 drehbar gelagertes zweites Betätigungselement 10.

Ein Hauptproblem bei der Herstellung derartiger medizinischer Instrumente 1 ist es, einerseits bei Beibehaltung aller Freiheitsgrade einen möglichst geringen Durchmesser des Schaftes 2 zu gewährleisten und andererseits einen Antriebsstrang 11 zur Weiterleitung der Bewegungen der beiden Betätigungselemente 9 und 10 zum Drehen der Werkzeugspitze 8 und zum Betätigen der Maulteile 5 und 6 vom Schaft 2 in die abwinkelbare Werkzeugspitze 8 so auszugestalten, dass dieser bei guter Reinigbarkeit eine gute Kraftübertragung auf das Werkzeug 4 ermöglicht.

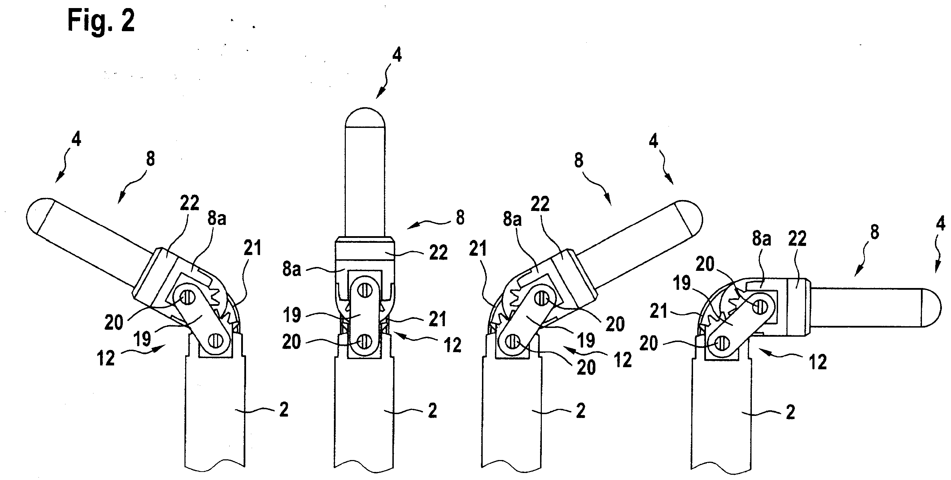

Die Abbildungen Fig. 2 bis Fig. 5 zeigen die Ausbildung eines Gelenks 12, über das die Werkzeugspitze 8 gegenüber dem Schaft 2 um bis zu 90° abwinkelbar ist.

Bei den meisten aus dem Stand der Technik bekannten medizinischen Instrumenten mit einer abwinkelbaren Werkzeugspitze erfolgt das Abwinkeln der Werkzeugspitze um eine starre Achse, wobei der Drehpunkt, um den die Werkzeugspitze gegenüber dem Schaft abwinkelbar ist, auf der Längsachse des Schaftes liegt. Diese Ausbildung des Abwinklungsgelenks bedingt jedoch, beim Abwinkeln der Werkzeugspitze zwangsläufig einen Längenversatz im Antriebsstrang zur Betätigung der Werkzeugspitze. Ohne eine Kompensation bewirkt dieser Längenversatz des Antriebsstrangs, dass beim Abwinkeln der Werkzeugspitze zwangsläufig auch die Maulteile des Werkzeugs zumindest geringfügig betätigt werden.

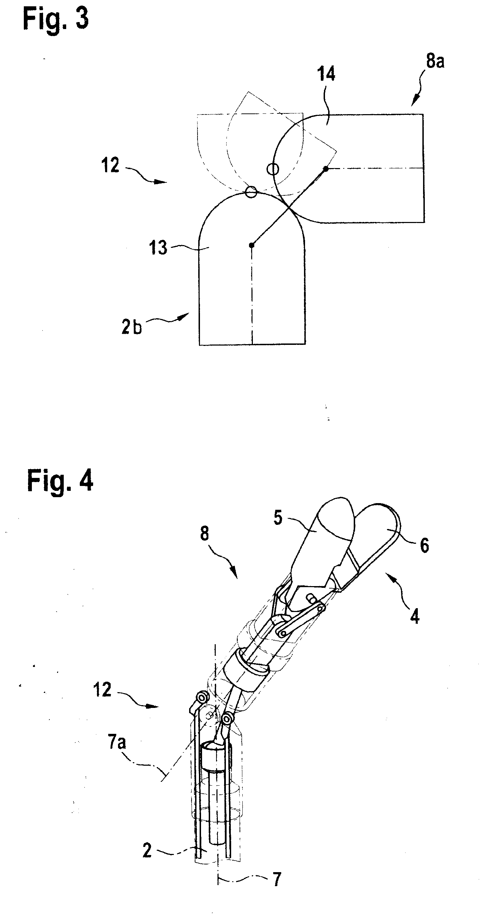

Das in den Abbildungen Fig. 2 bis 5 dargestellte Gelenk 12 zum Abwinkeln der Werkzeugspitze 8 ist so ausgebildet, dass das distale Ende 2b des Schaftes 2 und das proximale Ende 8a der Werkzeugspitze 8 kreissegmentförmig ausgebildet sind und die beiden Kreissegmente 13 und 14 zum Abwinkeln der Werkzeugspitze 8 aufeinander abrollen, wie dies insbesondere der Schemaskizze gemäß Fig. 3 zu entnehmen ist. Bei diesem Abrollen der Kreissegmente 13 und 14 aufeinander tritt kein Längenversatz des Antriebsstrangs 11 auf. Dies bedeutet, dass das Gelenk 12 des Antriebsstrangs 11 auch beim Abwinkeln der Werkzeugspitze 8 in derselben Position relativ zum Schaft 2 und zur Werkzugspitze 8 verbleibt, also auch keine zwangsweise Betätigung der Maulteile 5 und 6 beim Abwinkeln der Werkzeugspitze 8 erfolgt.

Beim Abwinkeln der Werkzeugspitze 8 wandert der Punkt, in welchem sich die Werkzeugspitze 8 und der Schaft 2 berühren, auf einem Kreisbogenabschnitt. Um die Werkzeugspitze 8 in einer definierten Lage relativ zum Schaft 2 zu halten, muss daher die Reibung zwischen der Werkzeugspitze 8 und dem Schaft 2 sehr hoch sein, oder aber ein Formschluss hergestellt werden.

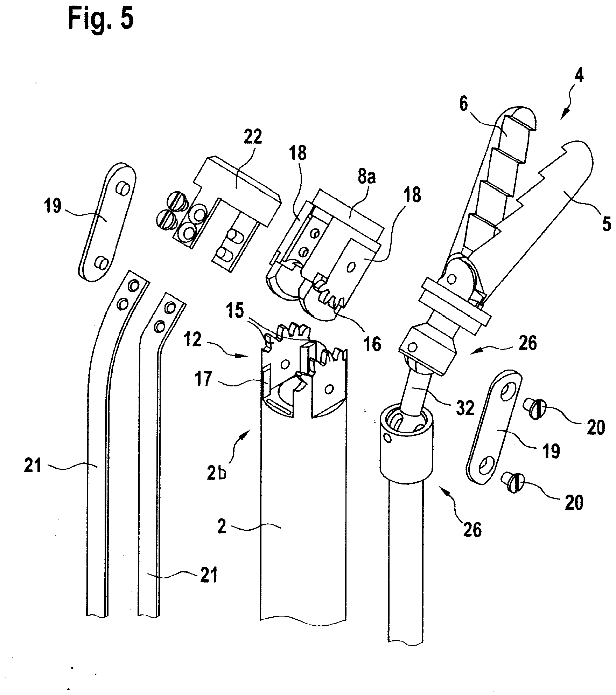

Wie aus der Abbildung Fig. 5 ersichtlich, sind bei der dargestellten Ausführungsform zur Ausbildung des Gelenks 12 zum Abwinkeln der Werkzeugspitze 8 die beiden Kreissegmente 13 und 14 als aufeinander abrollende Zahnradprofile 15 und 16 ausgebildet. Der zwischen den beiderseitigen Zähnen auftretenden Form- und Reibschlusses ermöglicht es, die Werkzeugspitze 8 in den unterschiedlichsten abgewinkelten Positionen zu halten.

Während bei der in Fig. 5 dargestellten Ausführungsform das proximale Ende 8a der Werkzeugspitze 8 als separates Bauteil ausgebildet, ist es selbstverständlich auch möglich, das proximale Ende 8a einstückig mit der Werkzeugspitze 8 auszubilden.

Zur Ausbildung der Zahnprofile 15 und 16 sind das distale Ende 2b des Schaftes 2 und das proximale Ende 8a der Werkzeugspitze 8 jeweils mit zwei parallel und mit Abstand zueinander angeordneten Stegen 17 und 18 versehen gabelförmig ausgebildet. Die Zahnradprofile 15 und 16 sind dabei an den freien Enden der Stege 17 und 18 des Schaftes 2 und der Werkzeugspitze 8 ausgebildet.

Durch die Verlagerung der das Gelenk 12 zum Abwinkeln der Werkzeugspitze 8 bildenden Zahnprofile 15 und 16 auf die seitlichen Stege 17 und 18 der gabelförmigen Enden verbleibt in der Mitte des Schaftes 2 und der Werkzeugspitze 8 ausreichend Platz für die Durchführung des Antriebsstrangs 11 für die Werkzeugspitze 8.

Wie weiterhin aus Fig. 2 und 5 ersichtlich, sind das distale Ende 2b des Schaftes 2 und das proximale Ende 8a der Werkzeugspitze 8 über zwei Laschen 19 miteinander verbunden, die in den Mittelpunkten der Zahnradprofile 15 und 16 mittels Schrauben 20 an den Stegen 17 und 18 festgelegt sind. Die Laschen 19 halten die beiderseitigen Zahnradprofile 15 und 16 in konstantem Abstand zueinander und gewährleisten so ein gleichmäßiges und im Wesentlichen spielfreies Abrollen der Zahnradprofile 15 und 16 aufeinander beim Abwinkeln der Werkzeugspitze 8.

Das Abwinkeln der Werkzeugspitze 8 gegenüber der Längsachse 7 des Schaftes 2 wird bei der dargestellten Ausführungsform gemäß Fig. 5 über zwei im Schaft 2 geführte und distalseitig an der Werkzeugspitze 8 festgelegte Zug-/Schubelemente 21 aktiviert. Das Festlegen der Zug-/Schubelemente 21 an der Werkzeugspitze 8 erfolgt bei dieser Ausführungsform über einen auf die Werkzeugspitze 8 aufsetzbaren Sicherungsring 22, der zusätzlich dazu dient, den Antriebsstrang 11 in axialer Richtung relativ zum Schaft 2 zu fixieren.

Proximalseitig stehen die Zug-/Schubelemente 21 mit der Handhabe 3 in Wirkverbindung. Vorteilhafterweise sind die Zug-/Schubelemente 21 als rechteckige Nitinolbänder ausgebildet. Die glatten Oberflächen der Bänder lassen sich gut reinigen und nutzen aufgrund ihrer rechteckigen Form den zur Verfügung stehenden Bauraum innerhalb des Schaftes 2 besser aus als beispielsweise kreisförmige Seilzüge.

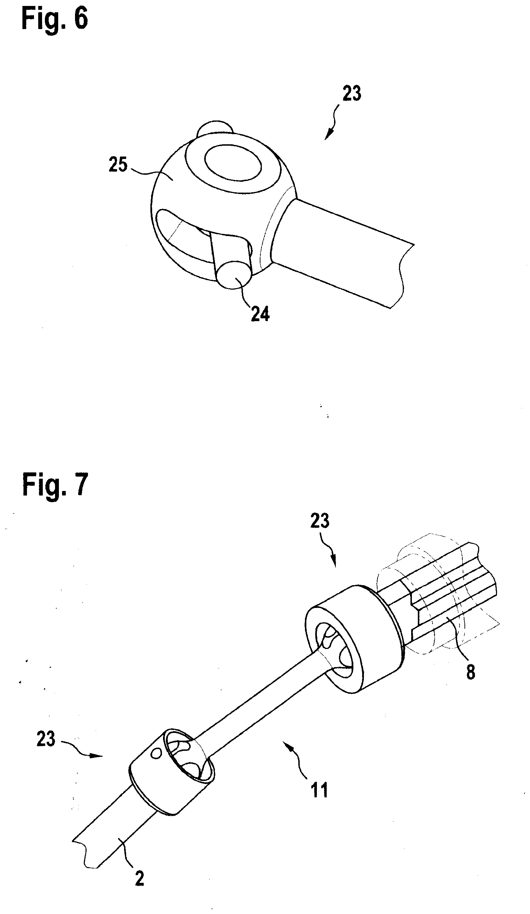

In den Abbildungen Fig. 6 bis Fig. 9 sind zwei Ausführungsbeispiele zur Ausbildung des Antriebstrangs 11 dargestellt, über den die Axial- und Drehbewegungen der im Schaft 2 gelagerten Betätigungselemente 9 und 10 in die abwinkelbare Werkzeugspitze 8 übertragen werden, um die Maulteile 5 und 6 des Werkzeugs 4 zwischen einer geöffneten und einer geschlossenen Position verstellen zu können und zusätzlich eine Rotation der Werkzeugspitze 8 um die Längsachse 7 des Schaftes 2 bzw. die Längsachse 7a der Werkzeugspitze 8 zu ermöglichen.

Um die Bewegungen der Betätigungselemente 9 und 10 in die um bis zu 90° abgewinkelte Werkzeugspitze 8 übertragen zu können, muss auch der Antriebsstrang 11 so ausgebildet sein, dass er die Dreh- und Schubbewegungen bei einer Abknickung des Antriebsstrangs 11 von ebenfalls bis zu 90° übertragen kann.

Bei der in Fig. 6 und 7 dargestellten ersten Ausführungsform zur Ausbildung des Antriebsstrangs 11, die nicht Gegenstand der Erfindung ist, besteht der Antriebsstrang 11 aus zwei in Reihe hintereinander geschalteten identischen Gelenken, die als kardanische CVD-Gelenke 23 ausgebildet sind.

Das Constant Velocity Drive CVD-Gelenk 23 basiert, ähnlich wie ein bekanntes Kardangelenk, auf einem Drehkreuz 24, welches jedoch im Gegensatz zum bekannten Kardangelenk nicht in gabelförmigen Aufnahmen der Achsstummel gelagert ist, sondern innerhalb einer ausgehöhlten Kugel 25 läuft. Da sich das CVD-Gelenk 23 innerhalb der ausgehöhlten Kugel 25 befindet, kann das CVD-Gelenk 23 aufgrund der Kugelform sehr hohe Kräfte aushalten. Das CVD-Gelenk 23 kann Schub- und Zugkräfte übertragen, während es um zwei Achsen gekippt wird.

Jedes CVD-Gelenk 23 für sich ermöglicht die Übertragung von Schub- und Zugbewegungen und Drehbewegungen bei einer Abwinklung des CVD-Gelenks 23 von bis zu 45°. Durch die Reihenschaltung von mindestens zwei identischen CVD-Gelenken 23 ist somit eine Abwinklung von mindestens 90° erzielbar.

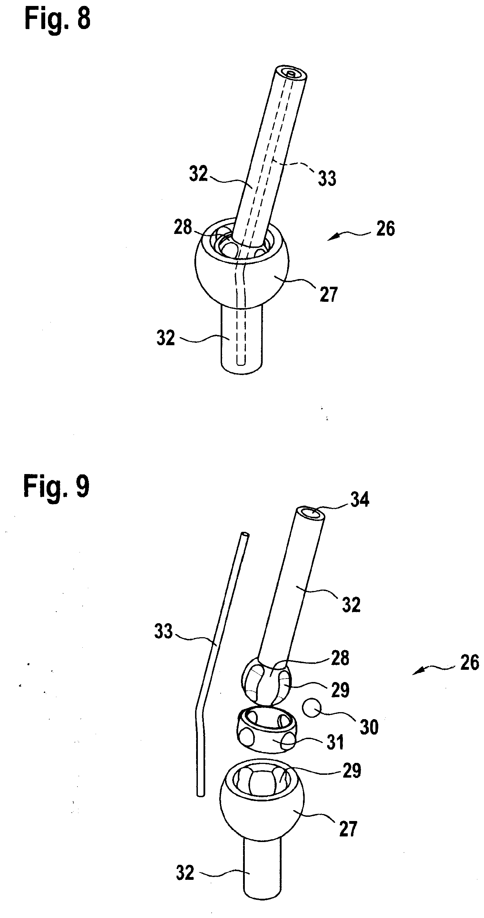

Gemäß der in Fig. 8 und 9 dargestellten alternativen zweiten Ausführungsform zur Ausbildung des Antriebsstrangs 11 besteht der Antriebsstrang 11 aus zwei in Reihe hintereinander geschalteten identischen Gelenken, die als Gleichlaufgelenke 26 ausgebildet sind.

Homokinetische oder auch gleichlaufende Gelenke zeichnen sich dadurch aus, dass sie eine gleichmäßige Winkelgeschwindigkeits- und Drehmomentübertragung von einer Welle auf eine winklig dazu angebrachte Welle ermöglichen. Die Gleichlaufgelenke 26 bestehen aus einer Kugelpfanne 27 und einem Kugelkopf 28, der in der Kugelpfanne 27 sitzt. Beide Bauteile 27, 28 weisen bis zu sechs Nuten 29 auf, die axial entlang der Kugeloberflächen von Kugelpfanne 27 und Kugelkopf 28 ausgebildet sind, wobei jeweils eine Nut 29 des Kugelkopfes 28 und eine Nut 29 der Kugelpfanne 27 ein Nutenpaar bilden, in welchem eine Kugel 30 zwangsgeführt wird. Zusätzlich werden alle Kugeln 30 eines jeden Gleichlaufgelenks 26 in einem gemeinsamen Kugelkäfig 31 geführt.

Jedes Gleichlaufgelenk 26 für sich ermöglicht die Übertragung eines Torsionsmoments bei einer Abwinklung zwischen Antriebs- und Abtriebsachse von bis zu 45°. Durch die Reihenschaltung von mindestens zwei identischen Gleichlaufgelenken 26 ist somit eine Abwinklung von mindestens 90° erzielbar. Axiale Kräfte sind mit den üblichen Gleichlaufgelenken 26 aufgrund der in Aixalrichtung verlaufenden Nuten 29 der Kugelbahnen nur in geringem Maße übertragbar.

Um die Übertragung von Axialkräften mittels eines Gleichlaufgelenks 26 deutlich zu verbessern, sind bei der dargestellten Ausführungsform ein Kugelkopf 28 und eine Kugelpfanne 27 eines jeden Gleichlaufgelenks 26 über einen im Inneren des Gleichlaufgelenks 26 angeordneten, in den Gelenkwellen 32 gelagerten Draht 33 miteinander verbunden sind. Eine axiale Bohrung 34 in der Kugelpfanne 27 und im Kugelkopf 28 führt den Draht 33, wobei der Draht 33 mit beiden Komponenten, Kugelpfanne 27 und Kugelkopf 28 verbunden ist.

Durch den das Gleichlaufgelenk 26 intern überbrückenden Draht 33 werden die Kugelpfanne 27 und der Kugelkopf 28 so zueinander fixiert, dass ohne Einschränkung der Beweglichkeit des Gleichlaufgelenks 26 nunmehr auch hohe axiale Kräfte mittels eines solchermaßen modifizierten Gleichlaufgelenks 26 übertragbar sind.

Ein wie zuvor beschrieben ausgebildetes medizinisches Instrument 1 zeichnet sich dadurch aus, dass dessen Gelenke 23, 26 bei möglichst vielen Freiheitsgraden und kleinem Bauraum eine hohe Kraftübertragung gewährleisten und dabei auch noch gut reinigbar sind.

### Bezuqszeichenliste

- 1: medizinisches Instrument
- 2: Schaft
- 2a: proximales Ende (Schaft)
- 2b: distales Ende (Schaft)
- 3: Handhabe
- 4: Werkzeug
- 5: Maulteil
- 6: Maulteil
- 7: Längsachse (Schaft)
- 7a: Längsachse (Werkzeugspitze)
- 8: Werkzeugspitze
- 8a: proximales Ende (Werkzeugspitze)
- 9: Betätigungselement
- 10: Betätigungselement
- 11: Antriebstrang
- 12: Gelenk
- 13: Kreissegment (Schaft)
- 14: Kreissegment (Werkzeugspitze)
- 15: Zahnprofil (Schaft)
- 16: Zahnprofil (Werkzeugspitze)
- 17: Steg (Schaft)
- 18: Steg (Werkzeugspitze)
- 19: Lasche
- 20: Schraube
- 21: Zug-/Schubelement
- 22: Sicherungsring
- 23: CVD-Gelenk
- 24: Drehkreuz
- 25: Kugel
- 26: Gleichlaufgelenk
- 27: Kugelpfanne
- 28: Kugelkopf
- 29: Nut
- 30: Kugel
- 31: Kugelkäfig
- 32: Gelenkwelle
- 33: Draht
- 34: Bohrung

## Patentansprüche

1. Medizinisches Instrument mit einem hohlen Schaft (2), an dessen proximalem Ende eine Handhabe (3) angeordnet ist und an dessen distalem Ende ein Werkzeug (4) mit zwei Maulteilen (5, 6) angeordnet ist, von denen mindestens ein Maulteil (5 oder 6) relativ zu dem anderen Maulteilen (6 oder 5) verschwenkbar ist, wobei ein das Werkzeug (4) tragender distaler Endbereich des Schaftes (2) als gegenüber der Längsachse (7) des Schaftes (2) abwinkelbare Werkzeugspitze (8) ausgebildet ist sowie die Werkzeugspitze (8) um die Längsachse (7) des Schaftes (2) bzw. um die Längsachse (7a) der Werkzeugspitze (8) drehbar ist und wobei das mindestens eine verschwenkbare Maulteil (5 oder 6) des Werkzeugs (4) zwischen einer geschlossenen und einer geöffneten Position verstellbar ist, wobei das Drehen der Werkzeugspitze (8) und das Betätigen der Maulteile (5, 6) über im Schaft (2) gelagerte Betätigungselemente (9, 10) erfolgt, die proximalseitig mit der Handhabe (3) gekoppelt sind,
**dadurch gekennzeichnet,**
**dass** zur Ausbildung eines Gelenks (12) zum Abwinkeln der Werkzeugspitze (8) das distale Ende des Schaftes (2) und das proximale Ende der Werkzeugspitze (8) kreissegmentförmig ausgebildet sind und die beiden Kreissegmente (13, 14) zum Abwinkeln der Werkzeugspitze (8) aufeinander abrollen und, dass die Weiterleitung der Bewegungen der beiden Betätigungselemente (9, 10) zum Drehen der Werkzeugspitze (8) und zum Betätigen der Maulteile (5, 6) vom Schaft (2) in die abwinkelbare Werkzeugspitze (8) über mindestens zwei in Reihe hintereinander geschaltete identische Gelenke (23, 26) erfolgt, die sowohl Dreh- als auch Schubbewegungen übertragen können, wobei die mindestens zwei in Reihe hintereinander geschalteten Gelenke als Gleichlaufgelenke (26) ausgebildet sind.

2. Medizinisches Instrument nach Anspruch 1, **dadurch gekennzeichnet, dass** zur Übertragung von Axialkräften ein Kugelkopf (28) und eine Kugelpfanne (27) eines jeden Gleichlaufgelenks (26) über einen im Inneren des Gleichlaufgelenks (26) angeordneten, in den Gelenkwellen (32) gelagerten Draht (33) miteinander verbunden sind.

3. Medizinisches Instrument nach Anspruch 2, **dadurch gekennzeichnet, dass** eine axiale Bohrung (34) in der Kugelpfanne (27) und im Kugelkopf (28) den Draht (33) führt.

4. Medizinisches Instrument nach Anspruch 2 oder 3, **dadurch gekennzeichnet, dass** der Draht (33) als Nitinoldraht ausgebildet ist.

5. Medizinisches Instrument nach Anspruch 1, **dadurch gekennzeichnet, dass** die beiden Kreissegmente (13, 14) als aufeinander abrollende Zahnradprofile (15, 16) ausgebildet sind.

6. Medizinisches Instrument nach Anspruch 1, **dadurch gekennzeichnet, dass** das distale Ende des Schaftes (2) und das proximale Ende der Werkzeugspitze (8) jeweils zwei parallel und mit Abstand zueinander angeordnete Stege (17, 18) aufweisend gabelförmig ausgebildet sind, wobei die Zahnradprofile (15, 16) an den freien Enden der Stege (17, 18) des Schaftes (2) und der Werkzeugspitze (8) ausgebildet sind.

7. Medizinisches Instrument nach Anspruch 6, **dadurch gekennzeichnet, dass** distale Ende des Schaftes (2) und das proximale Ende der Werkzeugspitze (8) über zwei Laschen (19) miteinander verbunden sind, die in den Mittelpunkten der Zahnradprofile (15, 16) an den Stegen (17, 18) festgelegt sind.

8. Medizinisches Instrument nach einem der Ansprüche 1, **dadurch gekennzeichnet, dass** das Abwinkeln der Werkzeugspitze (8) über zwei im Schaft (2) geführte und distalseitig an der Werkzeugspitze (8) festgelegte Zug-/Schubelemente (21) aktuiert wird.

9. Medizinisches Instrument nach Anspruch 8, **dadurch gekennzeichnet, dass** die Zug-/Schubelemente (21) als Nitinolbänder ausgebildet sind.

## Claims

1. Medical instrument with a hollow shaft (2), at the proximal end of which a handle (3) is arranged, and at the distal end of which a tool (4) with two jaw parts (5, 6) is arranged, of which at least one jaw part (5 or 6) is pivotable relative to the other jaw part (6 or 5), wherein a distal end region of the shaft (2) that carries the tool (4) is configured as a tool tip (8) that can be deflected with respect to the longitudinal axis (7) of the shaft (2), and the tool tip (8) is rotatable about the longitudinal axis (7) of the shaft (2) respectively about the longitudinal axis (7a) of the tool tip (8), wherein the at least one pivotable jaw part (5 or 6) of the tool (4) is adjustable between a closed and an open position, and wherein the rotation of the tool tip (8) and the actuation of the jaw parts (5, 6) take place via actuating elements (9, 10) which are mounted in the shaft (2) and are coupled at the proximal end to the handle (3),
**characterized in that**,
in order to configure a joint (12) for the deflection of the tool tip (8), the distal end of the shaft (2) and the proximal end of the tool tip (8) are configured in the shape of segments of a circle, and the two circle segments (13, 14) roll on each other for the deflection of the tool tip (8), and **in that** the movements of the two actuating elements (9, 10), for rotating the tool tip (8) and for actuating the jaw parts (5, 6), are conveyed from the shaft (2) into the deflectable tool tip (8) via at least two identical joints (23, 26) which are connected in series one behind the other and which can transmit rotation movements and also translatory movements, wherein the at least two joints connected in series one behind the other are configured as homokinetic joints (26).

2. Medical instrument according to Claim 1, **characterized in that**, in order to transmit axial forces, a ball head (28) and a ball socket (27) of each homokinetic joint (26) are connected to each other via a wire which is arranged in the interior of the homokinetic joint (26) and is mounted in the cardan shafts (32).

3. Medical instrument according to Claim 2, **characterized in that** an axial bore (34) in the ball socket (27) and in the ball head (28) guides the wire (33).

4. Medical instrument according to Claim 2 or 3, **characterized in that** the wire (33) is configured as a nitinol wire.

5. Medical instrument according to Claim 1, **characterized in that** the two circle segments (13, 14) are configured as gearwheel profiles (15, 16) that roll on each other.

6. Medical instrument according to Claim 1, **characterized in that** the distal end of the shaft (2) and the proximal end of the tool tip (8) are each configured with a fork shape, having two webs (17, 18) arranged at a distance from and parallel to each other, wherein the gearwheel profiles (15, 16) are configured at the free ends of the webs (17, 18) of the shaft (2) and of the tool tip (8).

7. Medical instrument according to Claim 6, **characterized in that** the distal end of the shaft (2) and the proximal end of the tool tip (8) are connected to each other by two brackets (19), which are fixed to the webs (17, 18) at the midpoints of the gearwheel profiles (15, 16).

8. Medical instrument according to one of Claims 1, **characterized in that** the deflection of the tool tip (8) is actuated via two pull/push elements (21) which are guided in the shaft (2) and are fixed at the distal end to the tool tip (8).

9. Medical instrument according to Claim 8, **characterized in that** the pull/push elements (21) are configured as nitinol bands.

## Revendications

1. Instrument médical comprenant une tige creuse (2), à l'extrémité proximale de laquelle est disposée une poignée (3) et à l'extrémité distale de laquelle est disposé un outil (4) comprenant deux parties de mâchoire (5, 6), dont au moins une partie de mâchoire (5 ou 6) est pivotante par rapport aux autres parties de mâchoire (6 ou 5), une zone d'extrémité distale de la tige (2) portant l'outil (4) étant conçue comme une pointe d'outil (8) qui peut être inclinée par rapport à l'axe longitudinal (7) de la tige (2) et la pointe d'outil (8) pouvant tourner sur l'axe longitudinal (7) de la tige (2) respectivement sur l'axe longitudinal (7a) de la pointe d'outil (8) et l'au moins une partie de mâchoire pivotante (5 ou 6) de l'outil (4) étant réglable entre une position fermée et une position ouverte, la rotation de la pointe d'outil (8) et l'actionnement des parties de mâchoire (5, 6) étant effectués par le biais d'éléments d'actionnement (9, 10) montés dans la tige (2) et accouplés à la poignée (3) du côté proximal,
**caractérisé en ce que**
pour former une articulation (12) destinée à incliner la pointe d'outil (8), l'extrémité distale de la tige (2) et l'extrémité proximale de la pointe d'outil (8) sont conçues en forme de segment de cercle et les deux segments de cercle (13, 14) roulent l'un sur l'autre pour incliner la pointe d'outil (8) et **en ce que** le transfert des mouvements des deux éléments d'actionnement (9, 10) pour faire tourner la pointe d'outil (8) et pour actionner les parties de mâchoire (5, 6) de la tige (2) jusque dans la pointe d'outil inclinée (8) est effectué par le biais d'au moins deux articulations identiques (23, 26) montées en série qui peuvent transmettre aussi bien le mouvement de rotation que le mouvement de poussée, les au moins deux articulations montées en série étant conçues comme des articulations homocinétiques (26).

2. Instrument médical selon la revendication 1, **caractérisé en ce que**, pour transmettre des forces axiales, une tête sphérique (28) et une cavité sphérique (27) de chaque articulation homocinétique (26) sont reliées l'une à l'autre par le biais d'un fil (33) disposé à l'intérieur de l'articulation homocinétique (26) et logé dans les arbres d'articulation (32).

3. Instrument médical selon la revendication 2, **caractérisé en ce qu'**un alésage axial (34), ménagé dans la cavité sphérique (27) et dans la tête sphérique (28), guide le fil (33).

4. Instrument médical selon la revendication 2 ou 3, **caractérisé en ce que** le fil (33) est conçu comme un fil de nitinol.

5. Instrument médical selon la revendication 1, **caractérisé en ce que** les deux segments de cercle (13, 14) sont conçus comme des profils de roue dentée (15, 16) qui roulent l'un sur l'autre.

6. Instrument médical selon la revendication 1, **caractérisé en ce que** l'extrémité distale de la tige (2) et l'extrémité proximale de la pointe d'outil (8) sont conçues en forme de fourche comportant chacune deux nervures (17, 18) parallèles et disposées à distance l'une de l'autre, les profils de roue dentée (15, 16) étant formés aux extrémités libres des nervures (17, 18) de la tige (2) et de la pointe d'outil (8).

7. Instrument médical selon la revendication 6, **caractérisé en ce que** l'extrémité distale de la tige (2) et l'extrémité proximale de la pointe de l'outil (8) sont reliées l'une à l'autre par deux pattes (19) qui sont fixées aux nervures (17, 18) au centre des profils de roue dentée (15, 16).

8. Instrument médical selon l'une des revendications 1, **caractérisé en ce que** l'inclinaison de la pointe d'outil (8) est actionnée par le biais de deux éléments de traction/poussée (21) qui sont guidés dans la tige (2) et fixés à la pointe d'outil (8) du côté distal.

9. Instrument médical selon la revendication 8, **caractérisé en ce que** les éléments de traction/poussée (21) sont conçus comme des bandes de nitinol.
